# EUROPÄISCHE PATENTSCHRIFT

(11) **EP 3 950 031 B1**
(45) Veröffentlichungstag und Bekanntmachung des Hinweises auf die Patenterteilung: **24.07.2024**
(21) Anmeldenummer: 21020479.8
(22) Anmeldetag: 27.11.2018
(51) Int. Cl.: A61M 16/00, A61M 16/04, A61M 16/12, A61M 16/14, A61M 16/16, A61M 16/20

(54) **SYSTEM ZUR ATEMGASVERSORGUNG**
SYSTEM FOR RESPIRATION GAS SUPPLY
SYSTÈME D'ALIMENTATION EN GAZ RESPIRATOIRE

(30) Priorität: 30.11.2017 DE 102017011088
(43) Veröffentlichungstag der Anmeldung: 09.02.2022
(62) Teilanmeldung aus: 18000922.7
(73) Patentinhaber: Löwenstein Medical Technology S.A., 2557 Luxembourg (LU)
(72) Erfinder: Schwaibold, Matthias, 76228 Karlsruhe (DE); Scheerer, Mario, 76530 Baden-Baden (DE); Schröter, Christof, 76307 Karlsbad (DE)
(74) Vertreter: Marx, Thomas

(56) Entgegenhaltungen:
- EP-A1- 2 181 726
- WO-A1-2011/068418
- US-A1- 2005 166 922
- US-A1- 2008 000 475
- US-A1- 2013 133 656
- US-A1- 2017 274 165

## Beschreibung

Die Erfindung ist in angehängten unabhängigen Anspruch 1 definiert. Die abhängigen Ansprüche stellen bevorzugte Ausgestaltungen der Erfindung dar. Die Offenbarung betrifft ein System zur Atemgasversorgung, umfassend eine Atemgasquelle, eine Steuereinheit, ein Drucksteuermodul und ein Flusssteuermodul, wobei ebenfalls eine Drucksensoreinrichtung, eine Flusssensoreinrichtung und eine Anwenderschnittstelle zur Vorgabe von Parametern der Atemgasversorgung oder zum Austausch von Daten vorgesehen sind. Die Steuereinheit aktiviert alternierend das Drucksteuermodul und das Flusssteuermodul, wobei das Drucksteuermodul die Atemgasquelle zur Vorgabe eines Atemgasdrucks ansteuert und wobei das Flusssteuermodul die Atemgasquelle zur Vorgabe eines Atemgasflusses ansteuert.

Bei der druckunterstützten und druckkontrollierten Beatmung wird der Atemantrieb des Patienten mehr oder weniger berücksichtigt. Sie ist im Gegensatz zur volumenkontrollierten Beatmung schonender, da hier keine hohen Spitzendrücke entstehen können. Wenn der Patient einatmet, wird der Atmungsvorgang unterstützt und die Atemarbeit dadurch erleichtert. Gekennzeichnet ist die druckunterstützte Beatmung durch ein unteres Druckniveau und ein oberes Druckniveau. Diese beiden Drücke sind variabel einstellbar. Aus der Menge an Luft, die bis Erreichen des oberen Druckes abgegeben wurde, ergibt sich das Atemzugvolumen. Hat der Patient längere Atempausen, kann er mittels einer Backup- oder Hintergrundfrequenz kontrolliert zwischenbeatmet werden. Die Triggerung der Inspiration und Exspiration kann fluss-, druck- oder zeitgesteuert erfolgen.

Bei einer CPAP-Therapie wird die Spontanatmung eines Patienten mit einem kontinuierlichen Überdruck unterstützt, um Atemstillstände zu vermeiden.

Bei der volumenkontrollierten Beatmungsform wird dem Patienten innerhalb einer vorgegebenen Inspirationszeit ein festgelegtes Atemvolumen appliziert. Das Atemminutenvolumen errechnet sich aus dem Atemzugvolumen und der Atemfrequenz. Bei dieser Beatmungsform können hohe Spitzendrücke entstehen, da das Beatmungsgerät in erster Linie versucht, das eingestellte Volumen zu applizieren. Um dieses Risiko zu verhindern, muss ein Maximaldruck als Alarmgrenze hinterlegt werden.

Mischformen der vorgenannten Beatmungsmodi versuchen die Vorteile der beiden Beatmungsformen Volumen- und Druckbeatmung zu vereinen. Es ist noch kein System zur Atemgasversorgung bzw. noch keine Atemgasquelle bekannt, die einen hohen Fluss vorgibt und alternierend dazu eine druckunterstützte oder druckkontrollierte Beatmung durchführt.

US 2013/133656 A1 offenbart ein Beatmungssystem, das abhängig davon, ob der Patient schläft oder wach ist, zwischen einem druckgesteuerten und einem flussgesteuerten Beatmungsmodus umschalten kann.

WO 2011/068418 A1 offenbart ein Beatmungsgerät, das einen Gasversorgungspfad zum Versorgen eines Patienten mit Atemgas aus Daten eines Sensors zum Messen von Parametern des Atemgases bestimmen kann.

US 2017/274165 A1 offenbart ein Beatmungsgerät zur flussgesteuerten Beatmung.

US 2008/000475 A1 offenbart ein Beatmungsgerät, das eine Cheyne-Stokes-Atmung anhand eines gemessenen Atemgasflusses erkennen und einen entsprechenden positiven exspiratorischen Druck bereitstellen kann.

Die Offenbarung betrifft daher ein System zur Atemgasversorgung, umfassend eine Atemgasquelle, eine Steuereinheit, einen Speicher, ein Drucksteuermodul, ein Flusssteuermodul, eine Drucksensoreinrichtung, eine Flusssensoreinrichtung und eine Anwenderschnittstelle zur Vorgabe von Parametern der Atemgasversorgung oder zum Austausch von Daten, mit einem Atemgasschlauch und einem Patienteninterface, wobei das System zusätzlich zumindest einen Anfeuchter und/oder eine Sauerstoffquelle und/oder einen Vernebler und/oder eine Heizung aufweist, wobei die Steuereinheit alternierend das Drucksteuermodul oder das Flusssteuermodul aktiviert, wobei das Drucksteuermodul die Atemgasquelle zur Vorgabe eines Atemgasdrucks ansteuert und wobei das Flusssteuermodul die Atemgasquelle zur Vorgabe eines Atemgasflusses ansteuert, wobei das Drucksteuermodul die Atemgasquelle zur Vorgabe eines niedrigen exspiratorischen Druckes und eines höheren inspiratorischen Druckes ansteuert.

Das System dient auch zum Mischen von Umgebungsluft (unter Fluss) und Sauerstoff (O₂).

Die Menge des zugeführten Atemgases wird über einen Flussmesser (1 bis 90 l/min) geregelt. Zusätzlich wird über einen Sauerstoffmischer die Applikation der gewünschten Sauerstoffmenge (bis zu 100 %) eingestellt.

Das Atemgas wird dem Patient über eine spezielle Nasenbrille oder Kanüle zugeführt, die zur Unterstützung der Spontanatmung dient.

Aufgrund des hohen Gasflusses entsteht ein positiver Atemwegsdruck (PEEP), der von der Höhe des Gasflusses abhängig ist. Durch den hohen Gasfluss wird Kohlendioxid aus dem Nasenrachenraum ausgewaschen.

Durch den hohen Gasfluss wird eine frühere Extubation (Entfernen des Beatmungsschlauchs) ermöglicht. Der hohe Gasfluss erleichtert die Atemarbeit. Der hohe Gasfluss minimiert die Atemanstrengung. Die Atemfrequenz wird durch hohe Flussraten reduziert.

Die Offenbarung betrifft auch eine Atemgasquelle. Die Atemgasquelle umfasst eine Steuereinheit, einen Speicher, ein Drucksteuermodul, ein Flusssteuermodul, eine Drucksensoreinrichtung, eine Flusssensoreinrichtung und eine Anwenderschnittstelle zur Vorgabe von Parametern der Atemgasversorgung oder zum Austausch von Daten, mit einem Atemgasschlauch und einem Patienteninterface, wobei das System zusätzlich zumindest einen Anfeuchter und/oder eine Sauerstoffquelle und/oder einen Vernebler und/oder eine Heizung aufweist. Die Atemgasquelle ist so ausgebildet, dass die Steuereinheit alternierend das Drucksteuermodul oder das Flusssteuermodul aktiviert, wobei das Drucksteuermodul die Atemgasquelle zur Vorgabe eines Atemgasdrucks ansteuert und wobei das Flusssteuermodul die Atemgasquelle zur Vorgabe eines Atemgasflusses ansteuert.

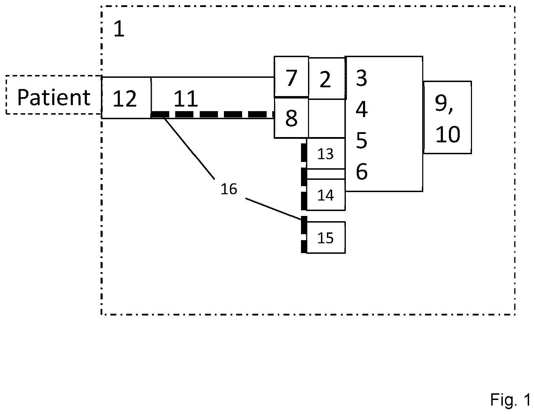

Figur 1 zeigt schematisch das System 1 zur Atemgasversorgung, umfassend eine Atemgasquelle 2, eine Steuereinheit 3, ein Drucksteuermodul 4, ein Flusssteuermodul 6 und einen Speicher 5, wobei ebenfalls eine Drucksensoreinrichtung 7, eine Flusssensoreinrichtung 8 und eine Anwenderschnittstelle 9 zur Vorgabe von Parametern 10 der Atemgasversorgung oder zum Austausch von Daten umfasst sind. Das System 1 weist einen Atemgasschlauch 11 und ein Patienteninterface 12 auf, wobei das System 1 zusätzlich zumindest einen Anfeuchter 13 und/oder eine Sauerstoffquelle 14 und/oder einen Vernebler 15 und/oder eine Heizung 16 aufweist. Die Steuereinheit 3 aktiviert alternierend das Drucksteuermodul 4 und das Flusssteuermodul 6, wobei das Drucksteuermodul 4 die Atemgasquelle 2 zur Vorgabe eines Atemgasdrucks im Bereich von 0 bis 90 mbar ansteuert und wobei das Flusssteuermodul 6 die Atemgasquelle 2 zur Vorgabe eines Atemgasflusses im Bereich von 0 bis 90 l/min ansteuert.

Die Steuereinheit 3 ist ausgebildet, um die Atemgasquelle 2 unter Verwendung des Drucksteuermoduls 4 oder des Flusssteuermoduls 6 zur automatischen Vorgabe eines Atemgasdruckes und/oder eines Atemgasflusses anzusteuern.

Die Steuereinheit 3 ist ausgebildet, um bei Aktivierung des Flusssteuermoduls 6 zusätzlich zumindest zeitweise den Anfeuchter 13 oder den Vernebler 15 und die Heizung 16 zur Erwärmung und Anfeuchtung des Atemgases zu aktivieren.

Bei Aktivierung des Flusssteuermoduls 6 kann die Steuereinheit 3 zusätzlich die Sauerstoffquelle 14 und/oder den Vernebler 15 zur Konditionierung des Atemgases aktivieren.

Der Atemgasschlauch 11 und/oder der Anfeuchter 13 und/oder der Vernebler 15 weisen jeweils die Heizung 16 auf. Alternativ weist zumindest der Atemgasschlauch 11 und/oder der Anfeuchter 13 und/oder der Vernebler 15 die Heizung 16 auf.

Die Steuereinheit 3 ist zur Erkennung von Atmungsbemühungen aus dem Drucksignal und/oder aus dem Flusssignal der Drucksensoreinrichtung 7 und/oder der Flusssensoreinrichtung 8 ausgebildet. Die Steuereinheit 3 ist zudem zur Erkennung von periodischer Atmung oder Atemaussetzern aus dem Drucksignal und/oder aus dem Flusssignal der Drucksensoreinrichtung 7 und/oder der Flusssensoreinrichtung 8 ausgebildet.

Die Anwenderschnittstelle 9 zur Vorgabe der Parameter 10 der Atemgasversorgung oder zum Austausch von Daten ist beispielsweise als ein Touchscreen ausgebildet. Alternativ oder ergänzend können auch ein Display und Eingabetasten vorgesehen sein. Ergänzend weist die Anwenderschnittstelle 9 zumindest eine Schnittstelle (USB, Bluetooth, WLAN ...) zur Verbindung mit externen Geräten oder einem Netzwerk auf, um Daten auszutauschen. Vorgegebene Parameter werden im Speicher 5 abrufbar abgelegt.

Die Atemgasquelle 2 ist beispielsweise als Elektromotor mit Lüfterrad oder als Druckgasquelle ausgebildet. Das Patienteninterface 12 ist als Nasenkanüle oder Maske oder als Tracheostomieanschluss ausgebildet.

Das System 1 ist so ausgebildet, dass bei Aktivierung des Flusssteuermoduls 6 als Patienteninterface 12 eine Nasenkanüle oder ein Tracheostomieanschluss verwendet wird und bei Aktivierung des Flusssteuermoduls 6 auch ein interner oder externer Anfeuchter 13 aktiviert und verwendet wird.

Das System 1 ist beispielsweise so ausgebildet, dass bei Aktivierung des Flusssteuermoduls 6 der interne Anfeuchter 13 deaktiviert wird. Stattdessen wird ein externer Anfeuchter 13 verwendet oder aktiviert, der leistungsstärker ist.

Das System 1 ist beispielsweise so ausgebildet, dass bei Aktivierung des Flusssteuermoduls 6 eine interne Sauerstoffquelle 14 deaktiviert wird. Stattdessen wird eine externe Sauerstoffquelle 14 verwendet.

Das System 1 ist beispielsweise so ausgebildet, dass bei Aktivierung des Flusssteuermoduls 6 eine Sauerstoffbeimischung über eine interne oder externe Sauerstoffquelle 14 bis 90 l/min erfolgt.

Das System 1 ist beispielsweise so ausgebildet, dass bei Aktivierung des Flusssteuermoduls 6 die Steuereinheit 3 unter Berücksichtigung der Signale der Flusssensoreinrichtung 8 und/oder der Drucksensoreinrichtung 7 die Sauerstoffquelle 14 zur Sauerstoffbeimischung aktiviert, die atemphasenabhängig moduliert wird. Beispielsweise ist daran gedacht, die Sauerstoffbeimischung so zu steuern, dass in Phasen der Inspiration dem Patienten mehr Sauerstoff zur Verfügung steht als zur Exspiration.

Das System 1 ist beispielsweise so ausgebildet, dass ein Pulsoximeter oder ein CO₂-Messgerät adaptiert werden kann, welches dann mit dem System 1 kommuniziert und/oder von ihm mit Energie versorgt wird. Über die Messwerte dieser Geräte kann der Anwender - oder ein automatischer Algorithmus - die richtige Flussrate und die richtige Sauerstoffbeimischung einstellen. Beispielsweise berücksichtigt die Steuereinheit 3 Messwerte des Pulsoximeters und/oder des CO₂-Messgerätes bei der Aktivierung oder Steuerung der Sauerstoffquelle 14 und/oder bei der Aktivierung oder Steuerung des Flusssteuermoduls 6.

Offenbarungsgemäß ist daran gedacht, dass der Anwender über die Anwenderschnittstelle 9 eine Vorgabe an die Steuereinheit 3 eingibt, um das Drucksteuermodul 4 oder das Flusssteuermodul 6 zu aktivieren. Die Vorgabe kann auch im Speicher 5 abgelegt sein und von der Steuereinheit 3 ausgelesen werden. Erfindungsgemäß ist auch daran gedacht, dass die Steuereinheit 3 selbsttätig anhand im Speicher 5 abgelegter Informationen das Modul 4 oder 6 wählt und unter Vorgabe der Parameter 10 (Druckwerte, Flusswerte ...) ansteuert.

Offenbarungsgemäß ist auch daran gedacht, dass die Steuereinheit 3 anhand einer Zeitvorgabe das Modul 4 oder 6 wählt und unter Vorgabe der Parameter 10 ansteuert. Offenbarungsgemäß ist auch daran gedacht, dass die Steuereinheit 3 ermittelt, welches Patienteninterface 12 adaptiert ist und anhand dieser Information das passende Modul aktiviert.

Offenbarungsgemäß ist auch daran gedacht, dass die Steuereinheit 3 ermittelt, welches Patienteninterface 12 adaptiert ist und abgleicht, ob das Patienteninterface 12 zum aktivierten Modul passt und entweder selbsttätig das passende Modul aktiviert oder einen Alarm aktiviert, um den Anwender auf eine inkompatible Kombination aus Patienteninterface 12 und Modul hinzuweisen.

Das System 1 erkennt beispielsweise, ob ein Ventilbeatmungsschlauch angeschlossen ist oder nicht. Das erkennt es daran, ob es in den Steuerleitungen für das Ventil einen Druck aufbauen bzw. messen kann. Wird ein Ventilbeatmungsschlauch verwendet, aktiviert die Steuereinheit 3 das Flusssteuermodul 6 nicht.

Offenbarungsgemäß ist auch daran gedacht, dass die Steuereinheit 3 das Flusssteuermodul 6 aktiviert und dabei bestimmt, welche Druckschwankungen im Schlauch durch die Atmung des Patienten ausgelöst werden. Sind diese größer als ein definierter Schwellenwert, ermittelt die Steuereinheit 3 daraus, dass das Patienteninterface 12 zu dicht oder ungeeignet ist. Es ist also beispielsweise eine Maske oder ein Tracheostomieanschluss adaptiert und keine Nasenkanüle. Es wird dann ein entsprechender Hinweis oder Alarm ausgegeben. Die Steuereinheit 3 könnte das Flusssteuermodul 6 dann auch deaktivieren.

Offenbarungsgemäß ist auch daran gedacht, dass die Steuereinheit 3 das Flusssteuermodul 6 aktiviert und dabei versucht, den eingestellten Fluss zu erzeugen, und dabei auch den dazu benötigten Druck bestimmt. Stößt dieser an eine vorgegebene Schwelle für einen Zeitraum von typischerweise ca. 0,001 bis 10 Sekunden, so schaltet die Steuereinheit 3 auf das Drucksteuermodul 4 um und hält einen vorgegebenen maximal erlaubten Druck, auch wenn der Fluss unter der eingestellten Vorgabe liegt. Wenn nun der eingestellte Fluss wieder erreicht oder überschritten, für einen Zeitraum von typischerweise ca. 0,001 bis 10 Sekunden, dann schaltet das Gerät wieder zurück auf das Flusssteuermodul 6.

Offenbarungsgemäß ist auch daran gedacht, dass die Steuereinheit 3 das Flusssteuermodul 6 aktiviert und dabei bestimmt, welche Atemphase vorliegt. Die Steuereinheit 3 erkennt Schwankungen durch die Atmung und versucht, diese kontinuierlich auszugleichen.

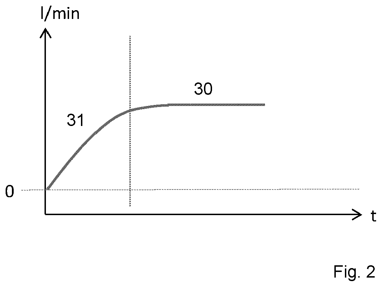

Figur 2 zeigt schematisch einen Flussverlauf 30, 31 des Systems 1 zur Atemgasversorgung. Die Atemgasquelle 2 wird hier von der Steuereinheit 3 unter Verwendung des Flusssteuermoduls 6 zur Vorgabe eines Atemgasflusses im Bereich von 0 bis 90 l/min angesteuert. Man erkennt, dass der Fluss 31 allmählich ansteigt und schließlich auf dem vorgegebenen Wert 30 konstant gehalten wird.

Die Flusssensoreinrichtung 8 ermittelt dabei den aktuellen Fluss und die Steuereinheit 3 gleicht diesen mit dem vorgegebenen Fluss ab und regelt den vorgegebenen Fluss 30 mit hoher Genauigkeit von zumindest +/- 5 % im Flussbereich von 5 bis 70 l/min ein. Die Vorgabe ist im Bereich von 0 bis 90 l/min, bevorzugt von 0 bis 60 l/min, in Schritten von beispielsweise 0,5 l/min möglich. Falls vom Anwender gewünscht, ist eine Sauerstoffbeimischung von 0 bis 90 l/min, bevorzugt von 1 bis 45 l/min, möglich. Dazu aktiviert die Steuereinheit 3 die Sauerstoffquelle 14. Zudem aktiviert die Steuereinheit 3 bei Verwendung des Flusssteuermoduls 6 immer die Heizung 16 und den Anfeuchter 13 oder den Vernebler 15 zur Konditionierung des Atemgases auf eine vorgebbare Temperatur und eine vorgebbare Feuchte. Diese Temperatur und diese Feuchte liegen typischerweise immer über der Temperatur und der Feuchte der Umgebungsluft. Die Temperatur ist im Bereich von 30° bis 37 °C, beispielsweise in 1-°C-Schritten, einstellbar.

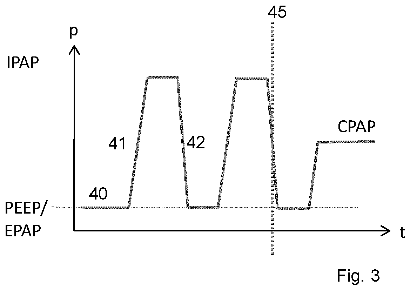

Figur 3 zeigt schematisch einen Druckverlauf 40, 41 ... des Systems 1 zur Atemgasversorgung. Die Atemgasquelle 2 wird hier von der Steuereinheit 3 unter Verwendung des Drucksteuermoduls 4 zur Vorgabe eines Atemgasdruckes im Bereich von 0 bis 90 mbar angesteuert. Man erkennt, dass der Druck von einem PEEP- oder EPAP-Niveau auf ein IPAP-Niveau ansteigt.

Der Übergang von PEEP oder EPAP auf IPAP kann in Form einer vorgebbaren Rampe 41 oder Wellenform erfolgen. Ebenso kann der Übergang von IPAP auf EPAP in Form einer vorgebbaren Rampe 42 oder Wellenform erfolgen. Der EPAP-Druck kann konstant sein oder erfindungsgemäß von einem initial geringen EPAP-Druck zum Ende der Exspiration ansteigen. Es ist erfindungsgemäß auch denkbar, dass der EPAP-Druck zumindest zwei unterschiedliche Druckniveaus umfasst.

Die Flusssensoreinrichtung 8 und/oder die Drucksensoreinrichtung 7 ermittelt dabei den aktuellen Fluss und/oder Druck und die Steuereinheit 3 ermittelt aus diesen Werten die Atemphase des Patienten und berücksichtigt diese zumindest zeitweise bei der Vorgabe der Drücke IPAP und EPAP. Die Steuereinheit 3 gleicht den Druck mit dem vorgegebenen Druck (aus dem Speicher 5) ab und regelt den Druck mit hoher Genauigkeit von zumindest +/-5 % im Bereich von 2 bis 45 mbar ein.

Figur 3 zeigt zudem, dass auf eine Anwenderauswahl 45 oder auf ein Ereignis 45 hin die Steuereinheit 3 den Druck auf einem konstanten CPAP-Niveau vorgibt. Das Ereignis erkennt die Steuereinheit 3 beispielsweise aus den Signalen der Flusssensoreinrichtung 8 und/oder der Drucksensoreinrichtung 7 als Atemaussetzer oder als periodische Atmung.

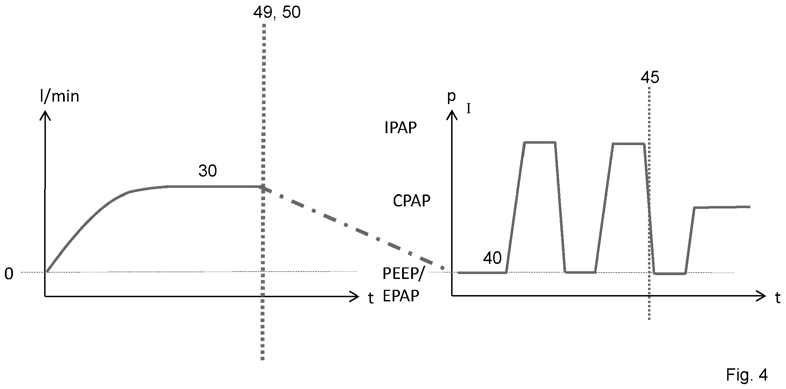

Figur 4 zeigt schematisch einen Flussverlauf 30 des Systems 1 zur Atemgasversorgung. Die Atemgasquelle 2 wird hier von der Steuereinheit 3 unter Verwendung des Flusssteuermoduls 6 zur Vorgabe eines Atemgasflusses im Bereich von 0 bis 90 l/min angesteuert. Figur 4 zeigt zudem schematisch einen Druckverlauf 40 des Systems 1 zur Atemgasversorgung. Die Atemgasquelle 2 wird hier von der Steuereinheit 3 unter Verwendung des Drucksteuermoduls 4 zur Vorgabe eines Atemgasdruckes im Bereich von 0 bis 90 mbar auf EPAP-, IPAP- oder CPAP-Druck angesteuert. Aus Figur 4 ist zudem ersichtlich, dass die Steuereinheit 3 alternierend das Flusssteuermodul 6 und dann das Drucksteuermodul 4 aktiviert. Der Übergang von der Flusssteuerung zur Drucksteuerung oder von der Drucksteuerung zur Flusssteuerung kann auf Anwenderauswahl 49 hin über die Anwenderschnittstelle 9 erfolgen oder automatisch auf ein Ereignis 50 hin unter Vorgabe der Steuereinheit 3 oder automatisch auf einen vorbestimmen Ablauf hin, der von der Steuereinheit 3 aus dem Speicher 5 gelesen wird. Ein solches Ereignis 50 kann der Wechsel der Tageszeit sein. Die Steuereinheit 3 weist dazu eine Uhr auf, die entsprechend vorgebbaren Zeitpunkten vom Tag- auf den Nacht-Modus umschaltet. Die Steuereinheit 3 kann den Wechsel vom Tag- auf den Nacht-Modus auf eine Anwendereingabe hin oder aufgrund einer Sensoreinrichtung, die den Schlaf des Anwenders feststellt, einleiten. Die Steuereinheit 3 kann das Flusssteuermodul 6 aktivieren, wobei dieses die Atemgasquelle 2 tagsüber zur Vorgabe eines Atemgasflusses ansteuert. Die Steuereinheit 3 registriert den Wechsel der Tageszeit und aktiviert nachts die Drucksteuerung zur Vorgabe eines Atemgasdruckes mit niedrigem exspiratorischem Druck und höherem inspiratorischem Druck oder auf einem CPAP-Niveau.

Das System 1 nach Fig. 4 ist auch so ausgebildet, dass die Steuereinheit 3 tagsüber das Flusssteuermodul 6 aktiviert, wobei das Flusssteuermodul 6 die Atemgasquelle 2 zur Vorgabe eines Atemgasflusses ansteuert und nachts zur Vorgabe zumindest eines Atemgasdruckniveaus, welches in Abhängigkeit von Signalen der Drucksensoreinrichtung 7 und/oder der Flusssensoreinrichtung 8 moduliert wird, wenn die Signale indikativ für periodische Atmung oder Atemaussetzer sind. Das Atemgasdruckniveau wird, wenn die Signale indikativ für periodische Atmung sind, so moduliert, dass die Druckvorgaben die periodische Atmung dämpfen oder verstärken. Das Atemgasdruckniveau wird, wenn die Signale indikativ für Atemaussetzer sind, so moduliert, dass die Druckvorgaben eine höhere Druckdifferenz zwischen EPAP und IPAP oder einen CPAP vorgeben.

Das offenbarungsgemäß High-Flow-System zur Atemgasversorgung baut durch einen Gasmischer einen Frischgasfluss von bis zu 90 l/min auf, wodurch der Durchfluss höher ist als bei Beatmungsgeräten. Durch den hohen Fluss strömt das Atemgas kontinuierlich zum Patienten, ohne dass dessen Eigenatmung behindert wird. Zum System gehört ein Gasmischer (Druckluft oder Lüfter und Sauerstoff), ein Atemwegsanfeuchter (der das Atemgas zugleich erwärmt), ggf. noch ein Reservoirbeutel und ein PEEP-Ventil. Der durch das PEEP-Ventil am Ende der Exspiration erzeugte Druck bleibt auch während der Inspiration erhalten, da während des gesamten Atemzyklus ein hoher Atemgasfluss vorhanden ist. Das System reduziert die Gefahr einer Druckinkonstanz unter Masken-CPAP.

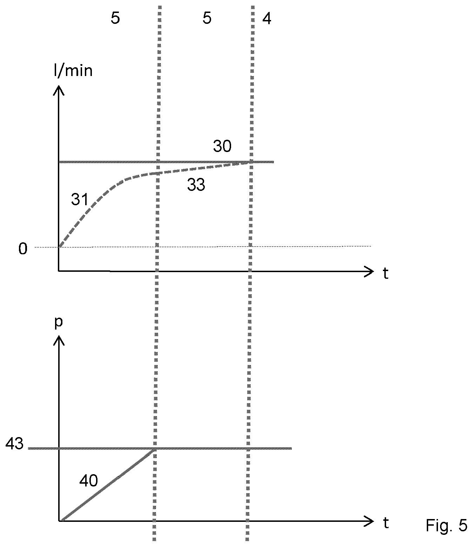

Das System 1 nach Fig. 5 ist auch so ausgebildet, dass die Steuereinheit 3 das Flusssteuermodul 6 aktiviert, wobei das Flusssteuermodul 6 die Atemgasquelle 2 zur Vorgabe eines vorgebbaren Atemgasflusses 30 ansteuert und in Abhängigkeit von Signalen der Drucksensoreinrichtung 7 und/oder der Flusssensoreinrichtung 8 den Druck erhöht, bis der vorgegebene Atemgasfluss erreicht ist. Das System 1 ist auch so eingerichtet, dass die Flusssensoreinrichtung 8 dabei den Atemgasfluss überwacht, wobei das Flusssteuermodul 6 anhand der Signale der Flusssensoreinrichtung 8 prüft, ob der vorgegebene Atemgasfluss erreicht ist, und wobei die Drucksensoreinrichtung 7 den Atemgasdruck überwacht, wobei das Flusssteuermodul 6 anhand der Signale der Drucksensoreinrichtung 7 prüft, ob ein vorgegebener maximaler Atemgasdruck 43 erreicht ist, und das Flusssteuermodul 6 den maximalen Atemgasdruck nicht weiter erhöht, bis der vorgegebene Atemgasfluss 30 erreicht ist. Dabei kann der aktuelle Fluss 33 für einige Sekunden oder Minuten unterhalb der Vorgabe 30 bleiben und sich ggf. nur langsam erhöhen. Wenn der vorgegebene Atemgasfluss 30 erreicht ist, kann eine Umschaltung auf das Flusssteuermodul 6 erfolgen.

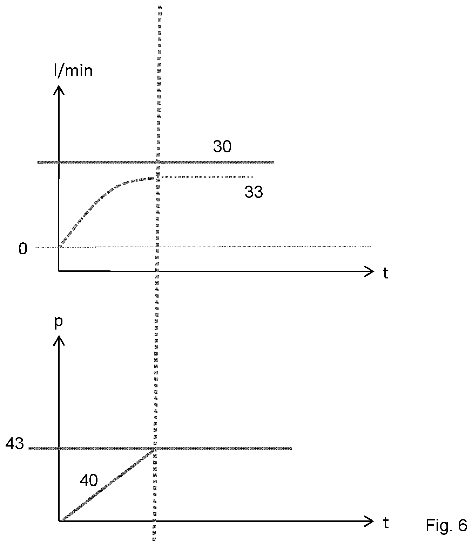

Das System 1 nach Fig. 6 ist auch so ausgebildet, dass die Steuereinheit 3 das Flusssteuermodul 6 aktiviert, wobei das Flusssteuermodul 6 die Atemgasquelle 2 zur Vorgabe eines vorgebbaren Atemgasflusses 30 ansteuert und in Abhängigkeit von Signalen der Drucksensoreinrichtung 7 und/oder der Flusssensoreinrichtung 8 den Druck erhöht, bis der vorgegebene Atemgasfluss 30 erreicht ist. Das System ist auch so eingerichtet, dass die Flusssensoreinrichtung 8 dabei den Atemgasfluss überwacht, wobei das Flusssteuermodul 6 oder die Steuereinheit 3 anhand der Signale der Flusssensoreinrichtung 8 prüft, ob der vorgegebene Atemgasfluss erreicht ist, und wobei die Drucksensoreinrichtung 7 den Atemgasdruck überwacht, wobei das Flusssteuermodul 6 oder die Steuereinheit 3 anhand der Signale der Drucksensoreinrichtung 7 prüft, ob ein vorgegebener maximaler Atemgasdruck 43 erreicht ist, und das Flusssteuermodul 6 oder die Steuereinheit 3 den maximalen Atemgasdruck nicht weiter erhöht, wenn der vorgegebene Atemgasfluss 30 nicht erreicht ist. Der maximale Atemgasdruck 43 wird auch dann nicht weiter erhöht, wenn der aktuelle Atemgasfluss 33 nicht die Vorgabe 30 erreicht.

## Patentansprüche

1. System (1) zur Atemgasversorgung, umfassend:
eine Atemgasquelle (2);
ein Drucksteuermodul (4) zum Ansteuern der Atemgasquelle (2) zur Vorgabe eines Atemgasdrucks mit einem exspiratorischen Druck (PEEP, EPAP) und einem im Vergleich zum exspiratorischen Druck (PEEP, EPAP) höheren inspiratorischen Druck (IPAP);
ein Flusssteuermodul (6) zum Ansteuern der Atemgasquelle (2) zur Vorgabe eines Atemgasflusses;
eine Drucksensoreinrichtung (7) zum Bereitstellen eines Drucksignals;
eine Flusssensoreinrichtung (8) zum Bereitstellen eines Flusssignals;
eine Anwenderschnittstelle (9) zur Vorgabe von Parametern (10) der Atemgasversorgung oder zum Austausch von Daten;
einen Speicher (5) zum Speichern der vorgegebenen Parameter (10);
eine Steuereinheit (3), die konfiguriert ist, um das Drucksteuermodul (4) oder das Flusssteuermodul (6) anhand einer über die Anwenderschnittstelle (9) von einem Anwender eingegebenen Vorgabe zu aktivieren oder anhand im Speicher (5) abgelegter Informationen selbsttätig zu wählen und unter Vorgabe der Parameter (10) anzusteuern, wobei die Steuereinheit (3) ferner konfiguriert ist, um aus dem Drucksignal und/oder dem Flusssignal Atemaussetzer zu erkennen,
wobei die Atemgasquelle (2) tagsüber zur Flusssteuerung und Vorgabe des Atemgasflusses und nachts zur Drucksteuerung und Vorgabe zumindest eines in Abhängigkeit vom Drucksignal und/oder vom Flusssignal modulierten Atemgasdruckniveaus angesteuert wird, wobei das Atemgasdruckniveau-wenn die Atemaussetzer erkannt wurden - so moduliert wird, dass die Druckvorgaben eine höhere Differenz zwischen EPAP und IPAP vorgeben, wobei die Steuereinheit (3) ferner konfiguriert ist, um beim Aktivieren des Flusssteuermoduls (6) zu versuchen, einen eingestellten Atemgasfluss zu erzeugen, wobei ein dazu benötigter Atemgasdruck bestimmt wird, wobei - wenn der dazu benötigte Atemgasdruck für einen bestimmten Zeitraum an eine vorgegebene Schwelle stößt - auf das Drucksteuermodul (4) umgeschaltet wird und ein vorgegebener maximal erlaubter Atemgasdruck gehalten wird, auch wenn der erzeugte Atemgasfluss unter dem eingestellten Atemgasfluss liegt, und - wenn der eingestellte Atemgasfluss für einen bestimmten Zeitraum wieder erreicht oder überschritten wird - zurück auf das Flusssteuermodul (6) umgeschaltet wird;
wobei das System (1) ferner einen Atemgasschlauch (11) und ein Patienteninterface (12) umfasst und zusätzlich zumindest einen Anfeuchter (13) und/oder eine Sauerstoffquelle (14) und/oder einen Vernebler (15) und/oder eine Heizung (16) aufweist,
**dadurch gekennzeichnet, dass** das EPAP-Niveau zumindest zwei Druckniveaus umfasst und von einem initial geringen EPAP zum Ende der Exspiration hin ansteigt.

2. System (1) nach Anspruch 1,
wobei das Drucksteuermodul (4) konfiguriert ist, um die Atemgasquelle (2) zur Vorgabe eines rampenförmigen Druckanstiegs von einem exspiratorischen Druckniveau auf ein inspiratorisches Druckniveau und zur Vorgabe eines rampenförmigen Druckabfalls vom inspiratorischen Druckniveau auf das exspiratorische Druckniveau anzusteuern.

3. System (1) nach einem der vorhergehenden Ansprüche,
wobei das Drucksteuermodul (4) konfiguriert ist, um die Atemgasquelle (2) zur Vorgabe des inspiratorischen Drucks mit einer vorgebbaren Druckwellenform und zur Vorgabe des exspiratorischen Drucks mit einer vorgebbaren Druckwellenform anzusteuern.

4. System (1) nach einem der vorhergehenden Ansprüche,
wobei das Drucksteuermodul (4) konfiguriert ist, um die Atemgasquelle (2) zur Vorgabe des inspiratorischen Drucks mit zwei unterschiedlichen inspiratorischen Druckniveaus und zur Vorgabe des exspiratorischen Drucks mit zwei unterschiedlichen exspiratorischen Druckniveaus anzusteuern.

5. System (1) nach einem der vorhergehenden Ansprüche,
wobei die Steuereinheit (3) konfiguriert ist, um die Atemgasquelle (2) zur Vorgabe eines vorgebbaren Atemgasflusses anzusteuern und in Abhängigkeit vom Drucksignal und/oder vom Flusssignal den Atemgasdruck zu erhöhen, bis der vorgegebene Atemgasfluss erreicht ist.

6. System (1) nach einem der vorhergehenden Ansprüche,
wobei die Flusssensoreinrichtung (8) konfiguriert ist, um den Atemgasfluss zu überwachen, und die Drucksensoreinrichtung (7) konfiguriert ist, um den Atemgasdruck zu überwachen, wobei die Steuereinheit (3) konfiguriert ist, um die Atemgasquelle (2) zur Vorgabe eines vorgebbaren Atemgasflusses anzusteuern und anhand des Flusssignals zu prüfen, ob der vorgegebene Atemgasfluss erreicht ist, und anhand des Drucksignals zu prüfen, ob ein vorgegebener maximaler Atemgasdruck erreicht ist, wobei die Steuereinheit (3) den maximalen Atemgasdruck nicht weiter erhöht, wenn der vorgegebene Atemgasfluss nicht erreicht ist.

7. System (1) nach einem der vorhergehenden Ansprüche,
wobei das System (1) ausgebildet ist, um die Menge des zugeführten Atemgases über einen Flussmesser zu regeln und die Applikation einer gewünschten Sauerstoffmenge über einen Sauerstoffmischer einzustellen.

8. System (1) nach einem der vorhergehenden Ansprüche,
wobei das System (1) ausgebildet ist, um beim Aktivieren des Flusssteuermoduls (6) eine interne Sauerstoffquelle (14) zu deaktivieren und eine externe Sauerstoffquelle zu verwenden.

9. System (1) nach einem der vorhergehenden Ansprüche,
wobei das System (1) so ausgebildet ist, dass ein Pulsoximeter oder ein CO₂-Messgerät adaptierbar ist, und ausgebildet ist, um mit dem Pulsoximeter oder dem CO₂-Messgerät zu kommunizieren und/oder das Pulsoximeter oder das CO₂-Messgerät mit Energie zu versorgen.

10. System (1) nach Anspruch 9,
wobei das System (1) ausgebildet ist, um eine Flussrate und eine Sauerstoffbeimischung mithilfe eines automatischen Algorithmus über Messwerte des Pulsoximeters und/oder des CO₂-Messgeräts einzustellen.

11. System (1) nach Anspruch 9 oder 10,
wobei die Steuereinheit (3) Messwerte des Pulsoximeters und/oder des CO₂-Messgeräts beim Aktivieren und/oder Steuern einer Sauerstoffquelle (14) und/oder des Flusssteuermoduls (6) berücksichtigt.

12. System (1) nach einem der vorhergehenden Ansprüche,
wobei das System (1) ausgebildet ist, um das Atemgasdruckniveau von einem PEEP- oder EPAP-Niveau in Form einer vorgebbaren Rampe oder Wellenform auf ein IPAP-Niveau anzuheben und vom IPAP- auf das EPAP-Niveau in Form einer vorgebbaren Rampe oder Wellenform abzusenken.

13. System (1) nach einem der vorhergehenden Ansprüche,
wobei die Steuereinheit (3) konfiguriert ist, um beim Aktivieren des Flusssteuermoduls (6) zu bestimmen, welche Atemphase vorliegt, und dabei Schwankungen durch die Atmung zu erkennen und zu versuchen, die Schwankungen kontinuierlich auszugleichen.

## Claims

1. A system (1) for supplying respiration gas, comprising:
a respiration gas source (2);
a pressure control module (4) for actuating the respiration gas source (2) to specify a respiration gas pressure at an expiratory pressure (PEEP, EPAP) and an inspiratory pressure (IPAP) that is higher than the expiratory pressure (PEEP, EPAP);
a flow control module (6) for actuating the respiration gas source (2) to specify a respiration gas flow;
a pressure sensor apparatus (7) for providing a pressure signal;
a flow sensor apparatus (8) for providing a flow signal;
a user interface (9) for specifying parameters (10) of the respiration gas supply or for exchanging data;
a memory (5) for storing the specified parameters (10);
a control unit (3) which is configured to activate the pressure control module (4) or flow control module (6) based on a specification input by a user via the user interface (9) or to automatically select said pressure control module or flow control module based on information stored in the memory (5) and to actuate said pressure control module or flow control module with specification of the parameters (10), wherein the control unit (3) is further configured to recognize interrupted breathing from the pressure signal and/or flow signal,
wherein the respiration gas source (2) is actuated during the day for flow control and specifying the respiration gas flow and during the night for pressure control and specifying at least one respiration gas pressure level that is modulated depending on the pressure signal and/or flow signal, wherein the respiration gas pressure level - if interrupted breathing was recognized - is modulated such that the pressure specifications specify a higher difference between EPAP and IPAP, wherein the control unit (3) is further configured to attempt to generate a set respiration gas flow when the flow control module (6) is activated, wherein a respiration gas pressure required for this is determined, wherein - if the respiration gas pressure required for this comes up against a specified threshold for a determined period of time - a switch takes place to the pressure control module (4) and a specified maximum permissible respiration gas pressure is maintained even if the generated respiration gas flow is below the set respiration gas flow, and - if the set respiration gas flow is again reached or exceeded for a determined period of time - a switch takes place back to the flow control module (6);
wherein the system (1) further comprises a respiration gas tube (11) and a patient interface (12) and, additionally, at least one humidifier (13) and/or an oxygen source (14) and/or a nebulizer (15) and/or a heater (16), **characterized in that** the EPAP level comprises at least two pressure levels and rises from an initially low EPAP up to the end of expiration.

2. The system (1) according to claim 1,
wherein the pressure control module (4) is configured to actuate the respiration gas source (2) to specify a ramp-shaped pressure increase from an expiratory pressure level to an inspiratory pressure level and to specify a ramp-shaped pressure drop from the inspiratory pressure level to the expiratory pressure level.

3. The system (1) according to any one of the preceding claims,
wherein the pressure control module (4) is configured to actuate the respiration gas source (2) to specify the inspiratory pressure with a specifiable pressure waveform and to specify the expiratory pressure with a specifiable pressure waveform.

4. The system (1) according to any one of the preceding claims,
wherein the pressure control module (4) is configured to actuate the respiration gas source (2) to specify the inspiratory pressure at two different inspiratory pressure levels and to specify the expiratory pressure at two different expiratory pressure levels.

5. The system (1) according to any one of the preceding claims,
wherein the control unit (3) is configured to actuate the respiration gas source (2) to specify a specifiable respiration gas flow and, depending on the pressure signal and/or flow signal, to increase the respiration gas pressure until the specified respiration gas flow has been reached.

6. The system (1) according to any one of the preceding claims,
wherein the flow sensor apparatus (8) is configured to monitor the respiration gas flow and the pressure sensor apparatus (7) is configured to monitor the respiration gas pressure, wherein the control unit (3) is configured to actuate the respiration gas source (2) to specify a specifiable respiration gas flow and, based on the flow signal, to check whether the specified respiration gas flow has been reached and, based on the pressure signal, to check whether a specified maximum respiration gas pressure has been reached, wherein the control unit (3) does not increase the maximum respiration gas pressure further if the specified respiration gas flow is not reached.

7. The system (1) according to any one of the preceding claims,
wherein the system (1) is designed to regulate the amount of supplied respiration gas via a flow meter and to set the application of a desired amount of oxygen via an oxygen mixer.

8. The system (1) according to any one of the preceding claims,
wherein the system (1) is designed to deactivate an internal oxygen source (14) when the flow control module (6) is activated and to use an external oxygen source.

9. The system (1) according to any one of the preceding claims,
wherein the system (1) is designed such that a pulse oximeter or a CO2 meter is adaptable, and is designed to communicate with the pulse oximeter or CO2 measuring device and/or to supply the pulse oximeter or CO2 measuring device with energy.

10. The system (1) according to claim 9,
wherein the system (1) is designed to set a flow rate and an oxygen admixture using an automatic algorithm via measured values of the pulse oximeter and/or the CO2 measuring device.

11. The system (1) according to claim 9 or 10,
wherein the control unit (3) takes account of measured values of the pulse oximeter and/or CO2 measuring device when an oxygen source (14) and/or the flow control module (6) is/are activated and/or controlled.

12. The system (1) according to any one of the preceding claims,
wherein the system (1) is designed to raise the respiration gas pressure level from a PEEP or EPAP level to an IPAP level in the form of a specifiable ramp or waveform and to lower the respiration gas pressure level from the IPAP to the EPAP level in the form of a specifiable ramp or waveform.

13. The system (1) according to any one of the preceding claims,
wherein the control unit (3) is configured, when the flow control module (6) is activated, to determine which breathing phase is present, and to recognize fluctuations through the breathing and to attempt to continuously compensate for the fluctuations.

## Revendications

1. Système (1) d'alimentation en gaz respiratoire, comprenant :
une source de gaz respiratoire (2) ;
un module de commande de pression (4) pour la commande de la source de gaz respiratoire (2) pour la spécification d'une pression de gaz respiratoire avec une pression expiratoire (PEEP, EPAP) et une pression inspiratoire (IPAP) supérieure à la pression expiratoire (PEEP, EPAP) ;
un module de commande d'écoulement (6) pour la commande de la source de gaz respiratoire (2) pour la spécification d'un flux de gaz respiratoire ;
un dispositif de capteur de pression (7) pour la mise à disposition d'un signal de pression ;
un dispositif de capteur d'écoulement (8) pour la mise à disposition d'un signal d'écoulement ;
une interface utilisateur (9) pour la spécification de paramètres (10) de l'alimentation en gaz respiratoire ou pour l'échange de données ;
une mémoire (5) pour l'enregistrement des paramètres spécifiés (10) ;
une unité de commande (3), laquelle est configurée pour activer le module de commande de pression (4) ou le module de commande d'écoulement (6) à l'aide d'une spécification entrée par un utilisateur par l'interface utilisateur (9), ou pour les sélectionner de façon autonome à l'aide d'informations enregistrées dans la mémoire (5) et pour les commander avec spécification des paramètres (10), l'unité de commande (3) étant en outre configurée pour détecter les arrêts respiratoires à partir du signal de pression et/ou du signal d'écoulement,
la source de gaz respiratoire (2) étant commandée de jour pour la commande de l'écoulement et la spécification de l'écoulement de gaz respiratoire, et de nuit pour la commande de la pression et la spécification d'au moins un niveau de pression de gaz respiratoire modulé en fonction du signal de pression et/ou du signal d'écoulement, le niveau de pression de gaz respiratoire étant modulé de telle sorte - lorsque les arrêts respiratoires ont été détectés - que les spécifications de pression spécifient une différence plus élevée entre EPAP et IPAP, l'unité de commande (3) étant en outre configurée pour essayer de générer un écoulement de gaz respiratoire défini, lors de l'activation du module de commande d'écoulement (6), une pression de gaz respiratoire requise à cet effet étant déterminée, le module de commande de pression (4) étant commuté - lorsque la pression de gaz respiratoire requise à cet effet pendant une période déterminée atteint un seuil spécifié -, et une pression de gaz respiratoire autorisée maximale spécifiée étant maintenue, également lorsque l'écoulement de gaz respiratoire généré est inférieur à l'écoulement de gaz respiratoire défini, et le module de commande d'écoulement (6) étant de nouveau commuté - lorsque l'écoulement de gaz respiratoire défini pour une période déterminée est de nouveau atteint ou dépassé ;
le système (1) comprenant en outre un tube de gaz respiratoire (11) et une interface patient (12) et présentant en outre au moins un humidificateur (13) et/ou une source d'oxygène (14) et/ou un nébuliseur (15) et/ou un chauffage (16), **caractérisé en ce que** le niveau EPAP comprend au moins deux niveaux de pression et augmente à partir d'un EPAP initialement faible à la fin de l'expiration.

2. Système (1) selon la revendication 1,
le module de commande de pression (4) étant configuré pour commander la source de gaz respiratoire (2) pour la spécification d'une augmentation de pression en forme de rampe d'un niveau de pression expiratoire à un niveau de pression inspiratoire et pour la spécification d'une chute de pression en forme de rampe du niveau de pression inspiratoire au niveau de pression expiratoire.

3. Système (1) selon l'une des revendications précédentes,
le module de commande de pression (4) étant configuré pour commander la source de gaz respiratoire (2) pour la spécification de la pression inspiratoire avec une forme d'onde de pression spécifiable et pour la spécification de la pression expiratoire avec une forme d'onde de pression spécifiable.

4. Système (1) selon l'une des revendications précédentes,
le module de commande de pression (4) étant configuré pour commander la source de gaz respiratoire (2) pour la spécification de la pression inspiratoire avec deux niveaux de pression inspiratoires différents et pour la spécification de la pression expiratoire avec deux niveaux de pression expiratoires différents.

5. Système (1) selon l'une des revendications précédentes,
l'unité de commande (3) étant configurée pour commander la source de gaz respiratoire (2) pour la spécification d'un écoulement de gaz respiratoire spécifiable et pour augmenter la pression de gaz respiratoire en fonction du signal de pression et/ou du signal d'écoulement jusqu'à ce que l'écoulement de gaz respiratoire spécifié soit atteint.

6. Système (1) selon l'une des revendications précédentes,
le dispositif de capteur d'écoulement (8) étant configuré pour surveiller l'écoulement de gaz respiratoire, et le dispositif de capteur de pression (7) étant configuré pour surveiller la pression de gaz respiratoire, l'unité de commande (3) étant configurée pour commander la source de gaz respiratoire (2) pour la spécification d'un écoulement de gaz respiratoire spécifiable et pour contrôler, à l'aide du signal d'écoulement, si l'écoulement de gaz respiratoire spécifié est atteint, et pour contrôler, à l'aide du signal de pression, si une pression de gaz respiratoire maximale spécifiée est atteinte, l'unité de commande (3) n'augmentant plus la pression de gaz respiratoire maximale lorsque l'écoulement de gaz respiratoire spécifié n'est pas atteint.

7. Système (1) selon l'une des revendications précédentes,
le système (1) étant conçu pour réguler, à l'aide d'un débitmètre, la quantité de gaz respiratoire amené et pour régler l'application d'une quantité d'oxygène souhaitée à l'aide d'un mélangeur d'oxygène.

8. Système (1) selon l'une des revendications précédentes,
le système (1) étant conçu pour désactiver une source d'oxygène interne (14) et pour utiliser une source d'oxygène externe, lors de l'activation du module de commande d'écoulement (6).

9. Système (1) selon l'une des revendications précédentes,
le système (1) étant conçu de telle sorte qu'un oxymètre de pouls ou qu'un appareil de mesure de CO₂ sont adaptables, et étant conçu pour communiquer avec l'oxymètre de pouls ou avec l'appareil de mesure de CO₂, et/ou pour alimenter en énergie l'oxymètre de pouls ou l'appareil de mesure de CO₂.

10. Système (1) selon la revendication 9,
le système (1) étant conçu pour régler un débit et un mélange d'oxygène à l'aide d'un algorithme automatique, par des valeurs de mesure de l'oxymètre de pouls et/ou de l'appareil de mesure de CO₂.

11. Système (1) selon la revendication 9 ou 10,
l'unité de commande (3) prenant en compte des valeurs de mesure de l'oxymètre de pouls et/ou de l'appareil de mesure de CO₂ lors de l'activation et/ou de la commande d'une source d'oxygène (14) et/ou du module de commande d'écoulement (6).

12. Système (1) selon l'une des revendications précédentes,
le système (1) étant conçu pour augmenter le niveau de pression du gaz respiratoire d'un niveau PEEP ou EPAP jusqu'à un niveau IPAP, sous la forme d'une rampe ou d'une forme d'onde spécifiables, et pour l'abaisser du niveau IPAP au niveau EPAP sous la forme d'une rampe ou d'une forme d'onde spécifiables.

13. Système (1) selon l'une des revendications précédentes,
l'unité de commande (3) étant configurée pour déterminer quelle phase respiratoire est présente, lors de l'activation du module de commande d'écoulement (6), et, ce faisant, pour détecter les fluctuations par la respiration et pour essayer de compenser les fluctuations en continu.
